(19) Europäisches Patentamt
European Patent Office
Office européen des brevets

(11) EP 2 380 489 A1

(12) EUROPÄISCHE PATENTANMELDUNG

(43) Veröffentlichungstag:
26.10.2011 Patentblatt 2011/43

(51) Int Cl.:
*A61B 5/00* (2006.01)

(21) Anmeldenummer: 10161071.5

(22) Anmeldetag: 26.04.2010

(84) Benannte Vertragsstaaten:
AT BE BG CH CY CZ DE DK EE ES FI FR GB GR
HR HU IE IS IT LI LT LU LV MC MK MT NL NO PL
PT RO SE SI SK SM TR
Benannte Erstreckungsstaaten:
AL BA ME RS

(71) Anmelder: Biotronik CRM Patent AG
6341 Baar (CH)

(72) Erfinder:
• Geistert, Wolfgang
79618, Rheinfelden (DE)

• Flach, Erhard
12305, Berlin (DE)
• Weiss, Ingo
12435, Berlin (DE)

(74) Vertreter: Lindner-Vogt, Karin L.
Biotronik SE & Co. KG
Woermannkehre 1
12359 Berlin (DE)

(54) Ableitvorrichtung und MRI-sicheres Kathetersystem

(57) Die Erfindung betrifft eine Ableitvorrichtung mit einem proximalen, außerhalb des Körpers befindlichen Ende und einem distalen Ende, insbesondere geeignet für lang gestreckte, temporär im Körper befindliche medizinische Instrumente, insbesondere elektrophysiologische Katheter oder temporäre Elektrodenleitungen gelöst, welches eine Ableithülle, die sich vom proximalen zum distalen Ende der Ableitvorrichtung erstreckt und ein Lumen, welches sich vom proximalen zum distalen Ende erstreckt und in welchem das Instrument verschieblich geführt werden kann, umfasst, wobei das Lumen von der Ableithülle umschlossen ist. Erfindungsgemäß zeichnet sich die Ableitvorrichtung dadurch aus, dass die Ableithülle zumindest teilweise aus elektrisch leitfähigem Material besteht und/oder ein die Ableithülle ein Ableitmittel bestehend aus elektrisch leitfähigem Material aufweist, so dass die Ableithülle dadurch ausgebildet ist, durch elektromagnetische Strahlung induzierte elektrische Energie abzuführen oder abzuleiten.

FIG. 5

EP 2 380 489 A1

**Beschreibung**

Die Erfindung betrifft eine Ableitvorrichtung für lang gestreckte, temporär im Körper befindliche medizinische Instrumente und ein System zur Behandlung des menschlichen Körpers bestehend aus einer Ableitvorrichtung und einem lang gestreckten medizinischen Instrument.

In den letzten Jahren haben nun Magnetresonanz-(MR)-Diagnosegeräte, im Folgenden auch MR-Geräte (zum Beispiel Magnet-Resonanz-Tomographen = MRT) genannt, wegen ihrer patientenschonenden, nichtinvasiven und völlig schmerz- sowie nebenwirkungsfreien Untersuchungsmethodik erheblich an Bedeutung gewonnen. Übliche medizinische Implantate aus leitenden Materialien, zu denen im Übrigen auch elektrophysiologische Katheter oder auch bekannte temporäre oder ständig implantierte intrakardiale Elektroden zu Interventionszwecken zählen, zeigen dabei die Problematik, dass sie sich in Magnetresonanz-Diagnosegeräten unter dem Einfluss der davon generierten elektromagnetischen Strahlung aufgrund elektromagnetischer Induktion und der Abgabe der induzierten Energie im Bereich ihrer Kontaktfläche (n) zum Gewebe - also beispielsweise Elektrodenpole, ausgeführt als Ring- und die Kopfelektroden - stark erwärmen. Der Grund hierfür liegt insbesondere in den massiven, metallischen Zuleitungen zu den Kontaktflächen, die als Antenne wirken und bei denen aufgrund ihrer Isolierung die durch Hochfrequenz-(HF)-Felder induzierten Antennenströme nur an den Kontaktflächen, die die elektrische Grenzfläche zum Gewebe bilden, in den Körperelektrolyten abgeleitet werden. Die erwähnten HF-Felder arbeiten beispielsweise in einem Arbeitsfrequenzbereich von 21 MHz bei einem 0,5-Tesla-MR-Tomographen. Der Arbeitsfrequenzbereich kann nach dem derzeitigen Stand der Technik bis zu 300 MHz bei 7-Tesla-MR-Tomographen reichen und liegt typischerweise beispielsweise bei 64MHz bei einem 1,5-Tesla-MR-Tomographen. Da eine extrem starke Erhitzung des Gewebes in der Nähe der Elektrodenpole auftreten kann, ist die Durchführung minimalinvasiver Katheterablationen unter Nutzung der Magnetresonanz-Bildgebung ausgeschlossen.

Um die gefährliche Erwärmung der Körperzellen zu verhindern bzw. zu minimieren, muss der maximale Antennenstrom limitiert bzw. reduziert werden. Vor allem auf dem Gebiet der intrakardialen Elektrodenleitungen schlagen bekannte Lösungen diskrete Bauelemente vor, welche als Bandsperre oder als Tiefpassfilter wirken und so für die interessierenden Frequenzen den Leitungswiderstand der Antenne limitieren. Andere Lösungen schlagen Kondensatoren vor, die parallel zur Isolation geschaltet sind und so den Antennenstrom ableiten.

Hierzu seien beispielsweise die US 6,944,489, die US 2003/0144720 A1, die US 2003/0144721 A1, die US 2005/0288751 A1 (und die im Wesentlichen Gleichlautenden, gleichzeitig veröffentlichten Parallel-Schriften US 2005/0288752 A1, US 2005/0288754 A1 und US 2005/0288756 A1) angeführt.

Prinzipiell besteht diese Möglichkeit, durch bauliche Maßnahmen auf die Induktivität und kapazitive Ankopplung der Antenne Einfluss zu nehmen und damit das Fließen des Antennenstromes zu vermindern, selbigen abzuleiten oder die Resonanzfrequenz zu verschieben. Die aus therapeutischer Sicht gestellten, baulichen Anforderungen an ein medizinisches Instrument der eingangs genannten Art lassen hierzu jedoch in der Regel nur wenig Spielraum.

Des Weiteren haben Antennen im Gegensatz zu der hier herangezogenen sehr vereinfachten Betrachtung auch weitere Resonanzfrequenzen, so dass die Verschiebung des Resonanzverhaltens der Elektrode dann ggf bei einem MR-Gerät mit anderen HF-Frequenzen die Resonanzbedingung wiederum erfüllt. Dieser Weg ist daher nicht vorteilhaft.

Als technologischer Hintergrund ist ferner die EP 0 884 024 B1 zu nennen, bei der ein Kondensator zwischen die Zuleitungen für den Ablationspol eines Katheters und einen ebenfalls daran angeordneten Messpol zur Aufnahme von EKG-Signalen geschaltet ist. Aufgrund dieses Kondensators kann sowohl Hochfrequenzenergie zur Ablation über die Ablationselektrode abgegeben werden als auch gleichzeitig eine EKG-Signalaufnahme erfolgen.

Ausgehend von diesem Stand der Technik liegt der Erfindung die Aufgabe zugrunde, ein medizinisches Instrument, insbesondere einen elektrophysiologischen Katheter oder eine temporäre implantierbare Elektrodenleitung ohne konstruktive Veränderungen auch in Strahlungsfeldern von MR-Diagnosegeräten ohne relevantes Risiko für den Träger einsetzbar zu machen. Insbesondere soll dies durch einfach und kostengünstig herstellbare Materialien geschehen, die eine gezielte effektive und ausreichende Verschlechterung der Eigenschaften der Elektrode hinsichtlich ihrer Antennencharakteristik ermöglichen, ohne dass es zu Stromkonzentrationen und entsprechend übermäßigen Erwärmungen an elektrischen Funktionsstellen des medizinischen Implantats, insbesondere an Kontaktstellen zum umgebenden Körper kommen kann.

Diese Aufgabe wird gemäß Anspruch 1 mit einer Ableitvorrichtung mit einem proximalen, außerhalb des Körpers befindlichen Ende und einem distalen Ende, insbesondere geeignet für lang gestreckte, temporär im Körper befindliche medizinische Instrumente, insbesondere elektrophysiologische Katheter oder temporäre Elektrodenleitungen gelöst, welches eine Ableithülle, die sich vom proximalen zum distalen Ende der Ableitvorrichtung erstreckt und ein Lumen, welches sich vom proximalen zum distalen Ende erstreckt und in welchem das Instrument verschieblich geführt werden kann, umfasst, wobei das Lumen von der Ableithülle umschlossen ist. Erfindungsgemäß zeichnet sich die Ableitvorrichtung dadurch aus, dass die Ableithülle zumindest teilweise aus elektrisch leitfähigem Material besteht und/oder die Ableithülle ein Ableitmittel bestehend aus elektrisch leitfähigem Material aufweist, so dass die Ableithülle dadurch ausgebildet ist, durch elektromagnetische Strahlung induzierte elektrische Energie abzuführen oder abzuleiten. In einer Ausführung könnte die Ableithülle beispielsweise eine Innenschicht (einen so genannter "liner") umfassen, der nichtleitend ist. Diese Innenschicht kann abschnittsweise vom proximalen zum distalen Ende verlaufen oder aber nur ein Stück am distalen oder proximalen Ende.

Durch diese einfache konstruktive Lösung wird - wie aus der späteren Schilderung des Hintergrundes der Erfindung im Rahmen der Beschreibung von Ausführungsbeispielen deutlich wird - erfindungsgemäß die Güte des Schwingkreises, den das medizinische Instrument mit dem Körper zusammen als Antenne bildet, durch die Ableitung soweit reduziert, dass einerseits die von der Antenne aufgenommene Energie vermindert wird und dass sich andererseits die Verluste in dem Gesamtgebilde Instrument/Isolation/Körper so verteilen, dass es zu keiner übermäßigen Stromkonzentration an bestimmten Punkten kommt.

Der wesentliche Vorteil der Verringerung der Güte der Antenne gegenüber einem Bandfilter besteht darin, dass die Wirkweise von der Resonanzfrequenz der Antenne und von der Frequenz des einstrahlenden elektromagnetischen Wechselfeldes unabhängig ist, d. h., die von der Antenne aufgenommene Energie wird bei allen Frequenzen vermindert. Auf diese Weise wirkt die erfindungsgemäße Lösung bei verschiedenen MR-Geräten mit verschiedenen Arbeitsfrequenzen gleichermaßen.

Es zeigen:

Fig. 1    ein schematisches Schaubild zur Darstellung des vereinfachten Funktionsprinzips einer Antenne als offener Schwingkreis,

Fig. 2    ein Ersatzschaltbild eines medizinischen Instruments als Schwingkreis mit ohmschen Verlusten,

Fig. 3    eine schematische Darstellung eines medizinischen Instruments wie einen elektrophysiologischen Katheter oder eine temporäre Elektrodenleitung mit Zuleitung und Elektrodenummantelung in seiner Körperumgebung,

Fig. 4    ein Diagramm zur Darstellung der Abhängigkeit der normierten Schwingkreisgüte von den ohmschen Widerstandsanteilen der Kapazität und Induktivität des Ersatzschaltbild-Schwingkreises gemäß Fig. 3,

Fig. 5    eine erste Ausführung der Ableitvorrichtung, ausgebildet als Sheath zur Energieableitung an das umliegende Körpergewebe,

Fig. 6    eine Ableitvorrichtung mit einem Anschlussmittel zur Ableitung der aufgenommenen Energie an einen externen Gegenpol,

Fig. 7    eine Ableitvorrichtung zur Ableitung der aufgenommenen Energie an einen externen Gegenpol mit mehreren Sheath(abschnitten).

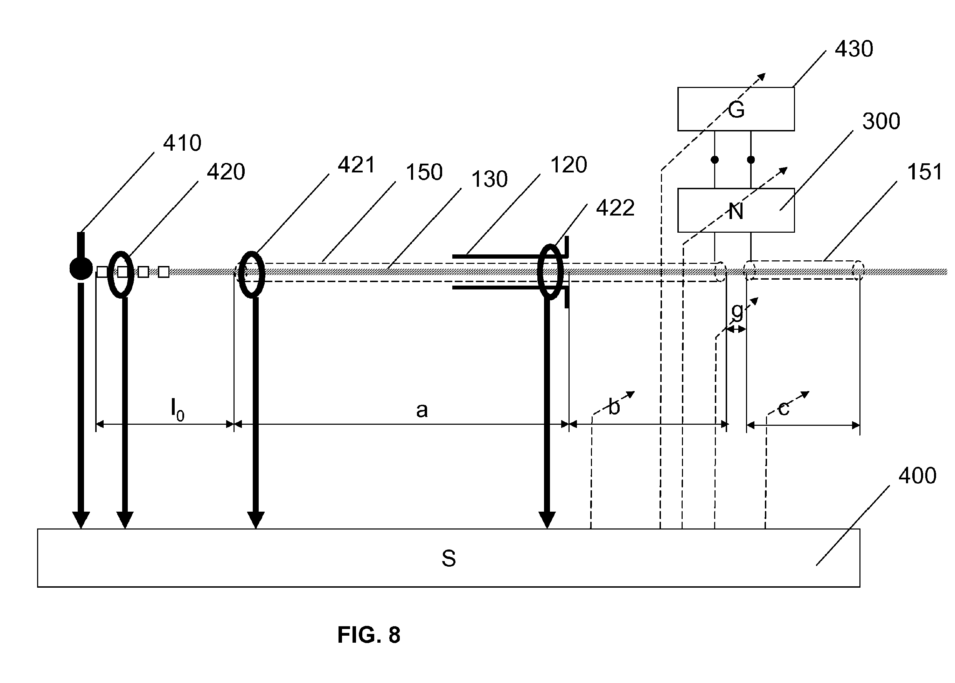

Fig. 8    eine Ableitvorrichtung mit einer Einheit zur Erfassung und Verarbeitung von Sensorsignalen.

Zum Hintergrund der Erfindung sollen erst die folgenden grundsätzlichen Ausführungen zum Schwingungsverhalten von Antennenkreisen gegeben werden:

Befindet sich ein vorzugsweise lang gestreckter elektrischer Leiter im Raum, wirkt dieser Leiter als Antenne für elektromagnetische Strahlung, die den Raum durchflutet. Dabei wandelt die Antenne die Freiraumwelle in eine Leitungswelle um, wodurch Antennenströme zum Fließen kommen. Diese Situation liegt beispielsweise vor, wenn ein Patient mittels minimalinvasiv einführbaren elektrophysiologischen Ablationskathetern in einem MR-Diagnosegerät behandelt wird. Die Ströme, die während dieser Behandlung in dem als Antenne wirkenden elektrophysiologischen Katheter entstehen, werden an den Kontaktpunkten zum Gewebe - in diesem Fall an den Elektrodenpolen - in den Körper geleitet und dort im Wesentlichen in Wärme umgewandelt. Je nach Stärke des elektromagnetischen Wechselfeldes kann das Ausmaß der Erwärmung für den Patienten gefährlich sein.

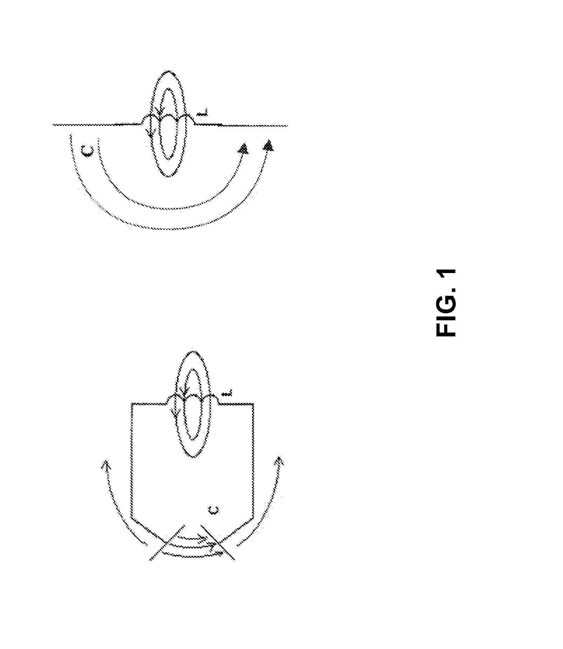

In vereinfachter Darstellung stellt eine Antenne einen offenen Schwingkreis dar, der so verstanden werden kann, als hätte man die Kondensatorplatten eines geschlossenen Schwingkreises in eine lang gestreckte Form auseinander gezogen (Fig. 1). In dieser vereinfachten Darstellung verkörpert der Antennenstab eine Induktivität L, deren Enden kapazitiv miteinander gekoppelt sind (Kapazität C). Der Antennenstrom erreicht seine maximale Amplitude, wenn Resonanz vorliegt, d. h. wenn der Schwingkreis auf der Resonanzfrequenz $f_0$ betrieben wird. Die Resonanzfrequenz des in Fig. 1 dargestellten Schwingkreises ist

$$f_0 = \frac{1}{2\pi\sqrt{LC}} \, .$$

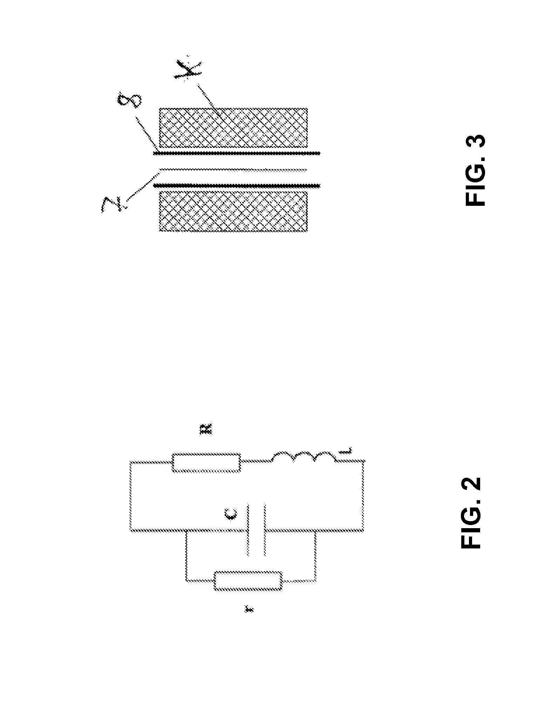

In Realität sind die Komponenten L und C verlustbehaftet, d. h. die Induktivität besitzt einen in Reihe geschalteten ohmschen Widerstand R, während der Kapazität ein ohmscher Widerstand r parallel geschaltet ist (siehe Fig. 2). Im Fall elektrophysiologischen Katheters, von dem ein Schaftausschnitt in Fig. 3 dargestellt ist, stellt eine Zuleitung 7 für beispielsweise Hochfrequenzsignale oder Messsignale den Antennenstab dar. Er weist eine von der Geometrie und den Materialeigenschaften abhängige Induktivität L und Leitungswiderstand R auf. Die kapazitive Kopplung der entfernten Leitungsabschnitte erfolgt über das umgebende dielektrische Medium (dazu gehören eine Katheterisolation 8 und Körper K des zu behandelnden Patienten). Ohmsche Verluste der Isolation und des Körpers sind im Ersatzschaltbild in Fig. 2 durch den Widerstand r zusammengefasst. Für die Funktion des elektrophysiologischen Katheters ist sowohl für die Ablation als auch für die Messung elektrophysiologischer Signale an der Behandlungsstelle anzustreben, dass die Zuleitung 7 in Längsrichtung möglichst gut leitet (d. h. R möglichst klein), die Isolation 8 hingegen aber sehr gut ist (d. h. r möglichst groß). Diese ohmschen Komponenten wirken sich auf die Güte Q des Schwingkreises aus, die ein Maß dafür ist, wie hoch die Antennenströme bei gegebener Intensität der elektromagnetischen Strahlung werden können. Die Güte berechnet sich als Quotient

$$Q = \frac{f_0}{B},$$

wobei $f_0$ die Resonanzfrequenz ist und die B die Bandbreite. Die Bandbreite ist durch die Grenzfrequenzen (f = f1, f = f2) definiert, außerhalb derer der Betrag der Leitfähigkeit $|\underline{Y}|$ unter $1/\sqrt{2}$ des Maximalwertes absinkt.

Im Falle des Schwingkreises nach Fig. 2 ist:

$$|\underline{Y}| = \left| \frac{1}{R + j2\pi fL + \frac{1}{1/r + j2\pi fC}} \right|, \quad (j = \sqrt{-1}).$$

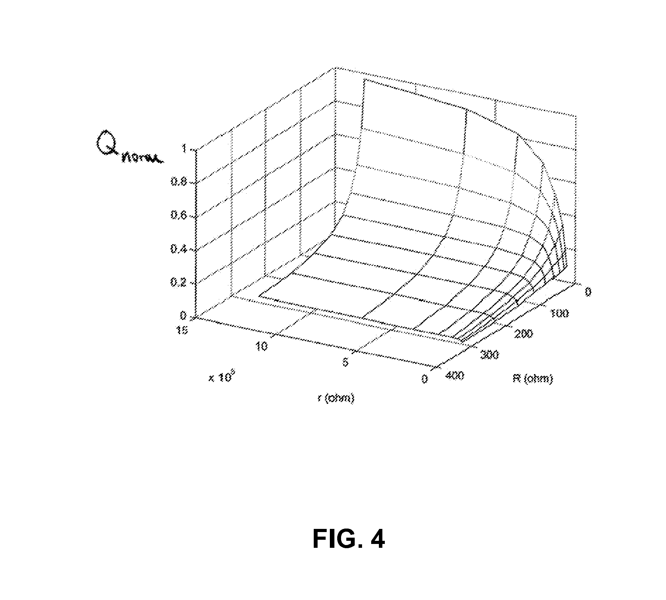

Die Güte des Schwingkreises (und damit auch der maximal mögliche Antennenstrom) ist für bekannte elektrophysiologische Katheter besonders gut, d. h. sie steigt mit sinkendem R und steigendem r. Dies wirkt sich besonders im Resonanzfall aus. Bedingt durch die physiologisch erforderte Länge des Katheters (von ca. 50 bis 130 cm), die dielektrischen Eigenschaften des Körpers und ferner durch die herkömmliche Bauweise tritt die Resonanzbedingung sehr nahe der Larmorfrequenz für Wasserstoff von ca. 64 MHz bei 1,5 T MR-Geräten auf. Mit beispielsweise einer Induktivität von typischerweise 3 µH der Elektrodenzuleitung und einer Kapazität von ca. 2 pF liegt die Resonanzfrequenz des betrachteten Schwingkreises bei $f_0$ = 64,97 MHz.
Zur Illustration veranschaulicht Fig. 4 die Abhängigkeit der Güte des verlustbehafteten Schwingkreises nach Fig. 2 von den ohmschen Verlustwiderständen R und r innerhalb praxisrelevanter Wertebereiche wobei L = 3 µH und C = 2 pF angenommen wurde. Qualitativ ist die Gestalt dieser Abhängigkeit auch für weit davon abweichende L und C Werte ähnlich.
Wie in Fig. 4 ersichtlich, kann die Güte der Antenne zweckmäßig verschlechtert werden, indem der Widerstand r der Antennenummantelung vermindert wird, der Widerstand R der Antennenleitung erhöht wird, oder beide Maßnahmen getroffen werden. Das heißt, es können die Antenneneigenschaften so beeinflusst werden, dass es in Strahlungsfeldern nicht zu Stromkonzentrationen und damit einhergehend zu übermäßigen Erwärmungen an den Elektrodenpolen kommen kann. So ist es bei intrakardialen Elektroden bekannt, dass diese Mittel umfassen, welche die Güte des Schwingkreises, den die Elektrode mit dem Körper zusammen als Antenne bildet, soweit reduziert, dass einerseits die von der Antenne aufgenommene Energie vermindert wird und dass sich andererseits die Verluste in dem Gesamtgebilde Elektrodenleiter/Isolation/Körper so verteilen, dass es zu keiner übermäßigen Stromkonzentrationen an bestimmten Punkten kommt.

So kann gemäß aus elektrophysiologischen Kathetern die übliche Elektrodenisolation einerseits durch ein minderisolierendes Material ersetzt werden, um so den Ummantelungswiderstand der Elektrodenzuleitung zum Körper hin zu reduzieren, die Güte der Antenne zu verschlechtern und die in die Elektrodenzuleitung induzierten Antennenströme und damit die verursachte Erwärmung in Gegenwart elektromagnetischer Wechselfelder zu minimieren.

Andererseits kann gemäß des Standes der Technik der Leitungswiderstand der Elektrodenzuleitung erhöht werden.

Um die Effektivität zu steigern, können die beiden soeben beschriebenen Lösungsansätze optimal kombiniert, d. h. jeweils sowohl der Ummantelungswiderstand des Elektrodenmantels verringert als auch der Leitungswiderstand der Elektrodenzuleitung erhöht werden, so dass gemäß Fig. 4 die Güte bestmöglich verringert wird. Besonders geeignet ist dieser Ansatz für Herzschrittmacher- und Neurostimulationselektroden, wie dies schon aus US 2006/0200218 A1 bekannt ist.

Beide Lösungen aus dem Gebiet der intrakardialen Elektrodenleitungen bieten sich bei medizinischen Instrumenten der eingangs genannten Art nicht immer an. Meist sind derartige Instrumente nicht ausgelegt, im großen Maße bauliche Änderungen zuzulassen. Dies kann sowohl größenbedingt sein als auch derart, dass es nicht möglich ist, beispielsweise Filtermittel zu integrieren. Beispielhaft für diesen Umstand seien elektrophysiologische Katheter genannt. Derartige Katheter müssen leicht steuerbar sein. Das heißt, es bietet sich wenig Platz, um beispielsweise den Leitungsquerschnitt zu erhöhen oder eine variable Dicke der Isolierung zu ermöglichen. Beides würde unweigerlich zu einer Versteifung des Schaftes führen und damit die Steuerbarkeit

Im Folgenden wird die Erfindung anhand eines elektrophysiologischen Katheters - im Folgenden auch Anwendungskatheter - als medizinisches Instrument beschrieben.

Bei der erfindungsgemäßen Lösung ist zusätzlich zum Anwendungskatheter ein äußerer Katheter (Ableitvorrichtung oder "Sheath") vorgesehen, welcher über den Anwendungskatheter geschoben wird.

Das erfindungsgemäße Sheath enthält jedoch eine Ableithülle oder ein Ableitmittel bestehend aus elektrisch leitfähigem Material, welche die Energieaufnahme aus dem HF-Feld durch lange, niederohmigen Strukturen des Anwendungskatheters verhindert oder reduziert. Bevorzugt kann die Ableithülle oder das Ableitmittel auch komplett aus elektrisch leitfähigem Material bestehen und ist dadurch ausgebildet, durch elektromagnetische Strahlung induzierte elektrische Energie abzuführen oder abzuleiten.

Die langen, niederohmigen Strukturen sind funktionsbedingt und können z. B. Drähte sein, die eine Verbindung von an oder in der Nähe der Katheterspitze befindlichen Polen oder Sensoren zu einem am proximalen Katheterende befindlichen Steckverbinder darstellen. Solche Strukturen können aber auch Metallgeflechte oder Metallprofile sein, welche der Versteifung, Formgebung oder Torsionsstabilisierung des Katheters dienen.

Die in der Ableithülle oder im Ableitmittel enthaltenen elektrisch leitfähigen Materialien könnten z. B. leitfähige Partikel oder Materialien sein, welche eine weitgehende Ableitung für die langen, niederohmigen Strukturen des Anwendungskatheters darstellen und/oder welche deren Antennenwirkung so verstimmen, dass diese für die im MRI-Scanner verwendeten HF-Frequenzen (64MHz beim 1,5T-Scanner, 128MHz beim 3T-Scanner,...) praktisch nicht mehr vorhanden ist. Bevorzugt ist das elektrisch leitfähige Material eine Polymermatrix, in welche leitfähige Partikel eingebettet sind, wobei die leitfähigen Partikel vorzugsweise eine lang gestreckte Form aufweisen, besonders bevorzugt mit einem Längen- zu Breiten- beziehungsweise Durchmesser-Verhältnis von größer 2, besonders bevorzugt von größer 100. Die Polymermatrix umfasst vorzugsweise Silikon, Polyurethan und/oder Peba (auch bekannt unter "Pebax") und die leitfähigen Partikel Carbon, besonders bevorzugt Carbon black und/oder Carbonfasern, Carbon-Nanopartikel, metallbeschichtete Carbonpartikel und/oder Carbon-Nanotubes, Leitruß und/oder Metallpartikel umfassen.

Gemäß einer anderen Ausführung kann das leitfähige Material der Ableithülle oder das Ableitmittel aus einem Metall, vorzugsweise einem biokompatiblen Metall, gebildet sein. Besonders bevorzugt ist das Ableitmittel ein Metallgeflecht, eine Metallwendel oder ein Metallprofil, wobei jedes dieser genannten Ableitmittel zumindest teilweise in die Ableithülle eingebettet ist. Das Material der Ableithülle solcher Geflecht-, Wendel- oder Profilschläuche kann gegebenenfalls nicht leitend sein.

In einer Ausführungsform können die Ableithülle und/oder das Ableitmittel mindestens abschnittsweise galvanisch oder kapazitiv mit dem die Ableitvorrichtung umgebenden Körpergewebe elektrisch gekoppelt sein, um die durch elektromagnetische Strahlung induzierte elektrische Energie an das umgebende Körpergewebe abzuführen oder abzuleiten. Die so aufgenommene Energie wird also von der Ableithülle oder dem Ableitmittel großflächig und ohne unerwünschte Wärmeentwicklung an das Körpergewebe abgeben. Dies kann beispielsweise durch ein teilweise freiliegendes Geflecht realisiert sein oder durch eine hohe und bis an die Oberfläche reichende Partikeldichte. Im Falle eines direkten, galvanischen Kontakts mit Körpergewebe müssen diese Materialien oder Partikel aus körperverträglichen Stoffen bestehen, wie Kohlenstoff, Gold, Platin, Palladium, Titan, Edelstahl oder dergleichen.

In einer Ausführungsform des Sheath' können die Ableithülle oder das Ableitmittel vom Körpergewebe isoliert, also elektrisch entkoppelt sein. In dieser Ausführungsform weist die Ableitvorrichtung am proximalen Ende ein Anschlussmittel auf, welches mit dem Ableitmittel elektrisch verbunden ist, um die durch elektromagnetische Strahlung induzierte elektrische Energie an einen außerhalb des Körpers befindlichen Gegenpol galvanisch oder kapazitiv abzuführen oder abzuleiten.

Die erfindungsgemäßen Sheath' werden, um ihre Funktion zu erfüllen, in der Regel so lang sein, dass sie den größten Teil des Anwendungskatheters bedecken. D. h. in der Regel wird nur der in der Behandlungsregion befindliche und meist steuerbare oder vorgeformte Teil des Anwendungskatheters aus dem Sheath herausschauen. Da dieser kürzer als ein Viertel der Wellenlänge der HF-Frequenz im MRI-Scanner ist, wirkt der aus dem Sheath herausschauende Teil des Anwendungskatheters nicht als Antenne für diese Frequenz.

Es ist jedoch wünschenswert, wenn das Sheath oder die Ableitvorrichtung in der Länge anpassbar ist, beispielsweise weil die Anwendungskatheter oder medizinischen Instrumente unterschiedlich lang sind oder weil eine größere Ableitstrecke entlang des Anwendungskatheters/medizinischen Instrumentes gewünscht ist. In diesem Falle umfasst die Ableithülle der Ableitvorrichtung einen ersten distalen Ableitabschnitt und einen zweiten proximalen Ableitabschnitt, wobei der zweite Ableitabschnitt relativ zum ersten Abschnitt verschieblich gelagert ist, um die Länge der Ableitvorrichtung und damit die Schutzlänge der Ableitvorrichtung so zu optimieren, dass die Schutzwirkung maximal ist. Um das einfache Verschieben und Handhaben auch in dieser Konfiguration zu gewährleisten, weist der zweite Ableitabschnitt eine Führung aufweist, welche den ersten Ableitabschnitt flüssigkeitsdicht verschieblich führt.

In der weiter oben genannten Konfiguration der erfindungsgemäßen Ableitvorrichtung, in der die vom MR-Diagnostikgerät aufgenommene Energie an einen externen Gegenpol abgeleitet wird, ist das Anschlussmittel am proximalen Ende des zweiten Ableitabschnittes, wobei dieses zweite Ableitmittel einen elektrischen Konnektor vorzugsweise an seinem distalen Ende aufweist, welcher geeignet ist, die vom ersten Ableitungsabschnitt aufgenommene Energie aufzunehmen und diese ebenfalls über das Anschlussmittel abzuleiten. Der Konnektor kann dabei beispielsweise ein Steckkontakt, Klemmkontakt, Schleifkontakt oder dergleichen, dem Fachmann bekannte Lösungen sein.

Der Gegenpol dient dazu, entweder die von der Ableitvorrichtung aufgenommene Energie ab- oder wegzuleiten oder durch andere geeignete Maßnahmen zu kompensieren. Deshalb ist der Gegenpol einerseits eine Vorrichtung zur Energieabfuhr, welche vorzugsweise die Schirmung der Kabine des MR-Diagnosegerätes, der Hochfrequenzschirm des Scanners, der Schirm eines elektrophysiologischen Ablationsgerätes oder das Potential der Neutralelektrode des elektrophysiologischen Ablationssystems. Möglich ist auch, dass es sich bei der Vorrichtung um eine Kombination der genannten externen Geräte ist. Zusätzlich oder alternativ zu den genannten Vorrichtungen kann das Anschlussmittel mit einem in Amplitude und Phase einstellbaren Hochfrequenzgenerator zur kompensierenden Energieeinkopplung elektrisch verbunden sein. Des Weiteren kann zwischen Anschlussmittel, Gegenpol und/oder Hochfrequenzgenerator ein Anpassungsnetzwerk bestehend aus RLC-Gliedern und/oder Übertragern vorgesehen ist, welches die Optimierung der Energieabfuhr oder -ableitung sicherstellt.

Gemäß eines weiteren Aspektes der Erfindung und in Anwendung der Ableitvorrichtung umfasst die Erfindung ein System zur Behandlung des menschlichen Körpers bestehend aus einer Ableitvorrichtung der vorgenannten Art und einem lang gestreckten, temporär im Körper befindlichen medizinischen Instrument. Dieses Instrument, was auch ein elektrophysiologischer Ablationskatheter oder aber eine temporäre kardiale Elektrode oder Nervenleitung sein kann weist einen in den menschlichen Körper einführbaren ersten Bereich mit einem distalen Ende und einem außerhalb des Körpers befindlichen zweiten Bereich mit einem proximalen Ende auf und umfasst mindestens eine galvanisch elektrisch leitende Struktur, wobei das medizinische Instrument in der Ableitvorrichtung verschieblich gelagert ist. Die Ableitvorrichtung zeichnet sich dadurch aus, dass es mindestens abschnittsweise den ersten Bereich des medizinischen Instrumentes umschließt. Der nicht umschlossene Abschnitt des medizinischen Instruments, also der Abschnitt, der sich zwischen distalen Ende des Instruments und dem distalen Ende der Ableitvorrichtung befindet, hat dabei eine Länge von kleiner als 1/4, vorzugsweise kleiner als 1/10 der Wellenlänge einer einstrahlenden Hochfrequenz-Welle — beispielsweise herrührend von einem MR-Diagnosegerät. Dies stellt einen optimalen Schutz des medizinischen Instrumentes dar, da die Antennenwirkung wie weiter oben beschrieben in einem solchen Abschnitt als besonders schlecht herausstellt. Da aber sichergestellt werden muss, dass das medizinische Gerät weiterhin seine Funktionalität hat, ist es von herausragender Bedeutung, dass die funktionellen Kontaktstellen eben in diesem Abschnitt sind.

Damit während der medizinischen Operation keine versehentlichen schutzfreien Abschnitte entstehen, kann die Ableitvorrichtung bevorzugt eine Arretierungseinrichtung umfassen, mit welcher das medizinische Instrument lösbar so arretiert werden kann, dass ein Verschieben des medizinischen Instruments in der Ableitvorrichtung verhindert wird.

Des Weiteren kann vorgesehen sein, dass das medizinische Instrument an oder in der Nähe einer seiner elektrisch leitenden Strukturen einen Temperatursensor enthält und dass Mittel vorgesehen sind, welche unter Berücksichtigung des von diesem Temperatursensor gelieferten Temperaturinformationen so auf den Hochfrequenzgenerator und/oder das Anpassungsnetzwerk einwirken, dass die Temperaturerhöhung über der Körpertemperatur minimiert wird. Dieser Temperatursensor oder die Mittel können auch so ausgebildet sein, dass sie dem Anwender Hinweise geben, wie die Länge der Ableitvorrichtung einzustellen ist, so dass die Temperaturerhöhung über der Körpertemperatur minimiert wird.

Die Erfindung wird im Folgenden anhand mehrerer Ausführungsbeispiele und Figuren beschrieben.

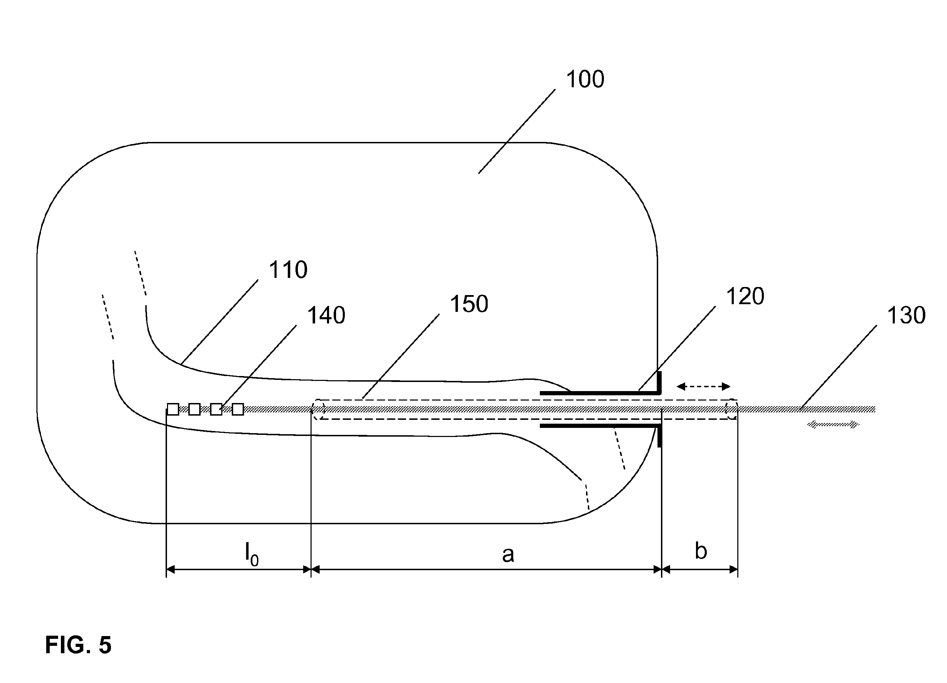

Fig. 5 zeigt eine erste einfache Ausführungsform der erfindungsgemäßen Ableitvorrichtung 150 mit und des erfindungsgemäßen Systems mit einem Anwendungskatheter 130 als medizinischem Instrument. In dieser Ausführung wird die durch das MR-Feld induzierte Energie an die Körperumgebung abgeführt. Das System ist in ein Körperlumen wie einem Blutgefäß 110 eines menschlichen oder tierischen Körpers 100 mittels einer Einführschleuse 120 eingeführt. In dieser Ausführungsform ist die Ableitvorrichtung 150 ein Sheath in Form eines im Wesentlichen zylinderförmigen und in der Regel flexiblen Schlauches (auch Ableithülle genannt) ausgeführt, durch dessen Lumen der Anwendungskatheter geführt wird.

Das erfindungsgemäße Sheath 150 befindet sich mit einer Länge a im Körper 100 bzw. im Körperlumen 110 und mit einer Länge b außerhalb des Körpers oder Blutgefäßes. Vorzugsweise ist b gleich 0. Das distale Ende des Anwendungskatheters 130 ragt mit den therapeutischen oder diagnostischen Katheterpolen 140 um eine Länge $1_0$ aus dem Sheath heraus. $1_0$ ist dabei vorzugsweise so gewählt, dass ein so groß wie möglicher Abschnitt des Anwendungskatheters 130 vom Sheath 150 abgedeckt, um so bei sicherer Anwendung des Katheters einen bestmöglichen Schutz vor Erwärmung erhält. Jedoch muss gewährleistet sein, dass $1_0$, also der nicht umschlossene Abschnitt des medizinischen Instruments zwischen seinem distalen Ende und dem distalen Ende der Ableitvorrichtung, kleiner als 1/4, vorzugsweise 1/10 der Wellenlänge einer einstrahlenden Hochfrequenz-Welle ist.

Der Anwendungskatheter 130 und das Sheath 150 sind unabhängig voneinander relativ zum Körper 100 verschiebbar. So kann eine Anpassung des Schutzbereiches, in dem der Sheath 150 den Anwendungskatheter 130 umschließt, optimal eingestellt werden. Um diese optimale Einstellung während der medizinischen Behandlung zu gewährleisten, umfasst das Sheath 150 eine Arretierungseinrichtung, mit dem es mit dem Katheter mechanisch verkoppelt werden kann, um die Länge $1_0$ gleichbleibend (und so kurz wie möglich) beizubehalten.

Gemäß einer weiteren bevorzugten Ausführungsform kann der Schlauch der Ableitvorrichtung aus einem ersten distalen Ableitabschnitt und einem zweiten proximalen Ableitabschnitt bestehen, wobei der zweite Ableitabschnitt relativ zum ersten Abschnitt verschieblich gelagert ist - beispielsweise durch eine Führung im zweiten Ableitungsabschnitt, welche den ersten Ableitabschnitt flüssigkeitsdicht verschieblich führt -, um die Länge der Ableitvorrichtung und damit die Schutzlänge der Ableitvorrichtung so zu optimieren, dass die Schutzwirkung maximal ist. Somit kann die Gesamtlänge des Sheath aus den Längen a und b mittels eines Teleskopsystems variabel gestaltet werden.

Das Ableitmittel im Schlauch des Sheath besteht (zumindest teilweise) aus elektrisch leitfähigen Materialien. Diese sind homogen oder heterogen in einer Trägermatrix verteilt (leitfähiger Kunststoff) eingebracht. Das elektrisch leitfähige Material kann dabei eine Polymermatrix sein, in welche leitfähige Partikel eingebettet sind, wobei die leitfähigen Partikel vorzugsweise eine lang gestreckte Form aufweisen, besonders bevorzugt mit einem Längen- zu Breiten- beziehungsweise Durchmesser-Verhältnis von größer 2, besonders bevorzugt von größer 100. Die Polymermatrix kann dabei vorzugsweise Silikon, Polyurethan und/oder Peba umfasst und die leitfähigen Partikel Carbon, bevorzugt Carbon black und/oder Carbonfasern, Carbon-Nanopartikel, metallbeschichtete Carbonpartikel und/oder Carbon-Nanotubes, Leitruß und/oder Metallpartikel umfassen. In einer weiteren Ausführung ist es auch möglich, den kompletten Sheath aus einem dieser leitfähigen Materialien zu erstellen.

Gemäß einer anderen Ausführungsform können die Ableitmittel aus metallischen Leiterbahnen, Geflechten, Wendeln, Profilen oder Schichten im Kunststoffschlauch eingebettet sein. Auch sind Metallisierungen (bzw. leitfähige Schichten) der Innen- oder/und Außenmantelfläche des Schlauches bestehen. In einer besonderen Ausführung ist die Schicht helixförmig aufgebracht. Die Ableithülle und/oder das Ableitmittel aller genannter Ausführungsformen sind mindestens abschnittsweise galvanisch oder kapazitiv mit dem die Ableitvorrichtung umgebenden Körpergewebe elektrisch gekoppelt, um die durch elektromagnetische Strahlung induzierte elektrische Energie an das umgebende Körpergewebe abzuführen oder abzuleiten.

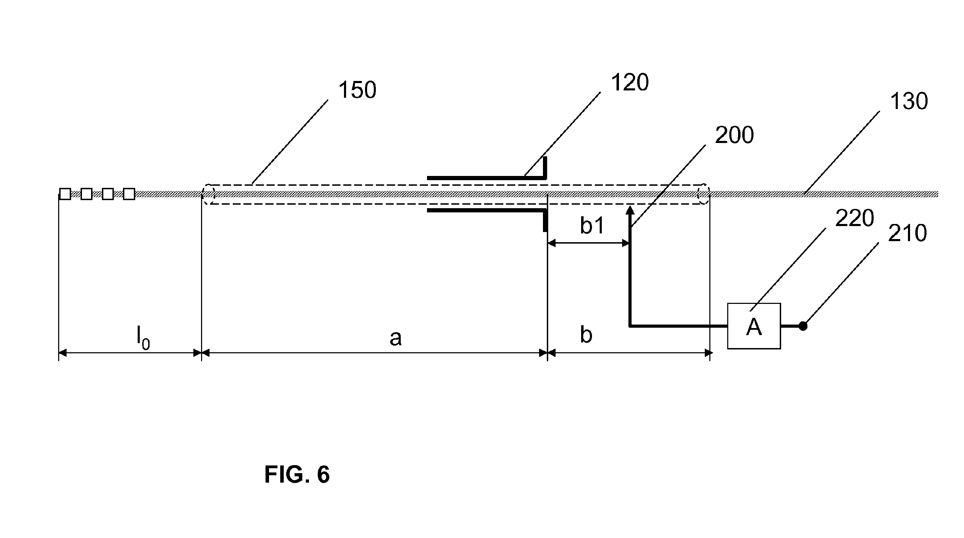

In Fig. 6 ist eine wie eben genannte Ableitvorrichtung bzw. ein System gezeigt. Die Bezeichnungen sind aus diesem Grunde identisch wie in Fig. 5. Die gezeigte Ableitvorrichtung 150 weist ein elektrisches Anschlussmittel 200 in Form eines elektrischen Abgriffes auf, welches entweder mit dem Ableitmittel im Schlauch oder - wenn der Schlauch selbst aus leitfähigem Material hergestellt ist - elektrisch verbunden ist, um die abgeleitete Energie außerhalb des Körper abzuleiten. Die Anschlussmittel können die Verbindung galvanisch wie beispielsweise durch Schleifkontakte, Steckverbindung und ähnlichem herstellen. Sie können aber auch eine kapazitive Kopplung bewirken. Optimalerweise befindet sich das Anschlussmittel 200 in einer Entfernung $b_1$ von der Schleuse entfernt. In einem Spezialfall ist $b_1$ gleich 0. Der Abgriff wird dann an bzw. mittels der Einführschleuse realisiert, welche dann elektrische Kontakte zur Kontaktierung der Ableitvorrichtung aufweist. In einer weiteren Realisierung ist der Abgriff stets am Ende des Sheath d.h. $b_1$ ist gleich b.

Das Anschlussmittel 200 stellt einen elektrischen Anschluss 210 zu einem außerhalb des Körpers liegenden Gegenpol her. Bei dem Gegenpol 210 kann es sich dabei um eine Vorrichtung zur Energieabfur wie beispielsweise die Schirmung der Kabine des MR-Diagnosegerätes, den Hochfrequenzschirm des Scanners, den Schirm eines elektrophysiologischen Ablationsgerätes, den Schirm der Filterbox zwischen Ablator und Patient oder das Potential der Neutralelektrode des elektrophysiologischen Ablationssystems handeln. Zusätzlich oder alternativ zum elektrischen Anschluss 210 an einen Gegenpol kann das Anschlussmittel 200 mit einem in Amplitude und Phase einstellbaren Hochfrequenzgenerator zur kompensierenden Energieeinkopplung elektrisch verbunden sein. Dies dient der kompensierenden Energieeinkopplung, wobei der Hochfrequenzgenerator ein hochfrequentes Signal erzeugt, das demjenigen des jeweiligen Scanners entspricht und in Amplitude und Phase einstellbar ist. Zur phasenstabilen Kopplung mit dem Hochfrequenzsystem des MR-Gerätes hat dieser Hochfrequenzgenerator eine Phasenregelschleife, auch als Phase-locked loop (PLL) bekannt.

Weiterhin kann die erfindungsgemäße Ableitvorrichtung zwischen seinem Anschlussmittel 200, dem elektrisch Anschluss 210 des Gegenpols und/oder des Hochfrequenzgenerators ein Anpassungsnetzwerk 220 bestehend aus RLC-Gliedern und/oder Übertragern vorsehen ist, welches die Optimierung der Energieabfuhr oder -ableitung sicherstellt.

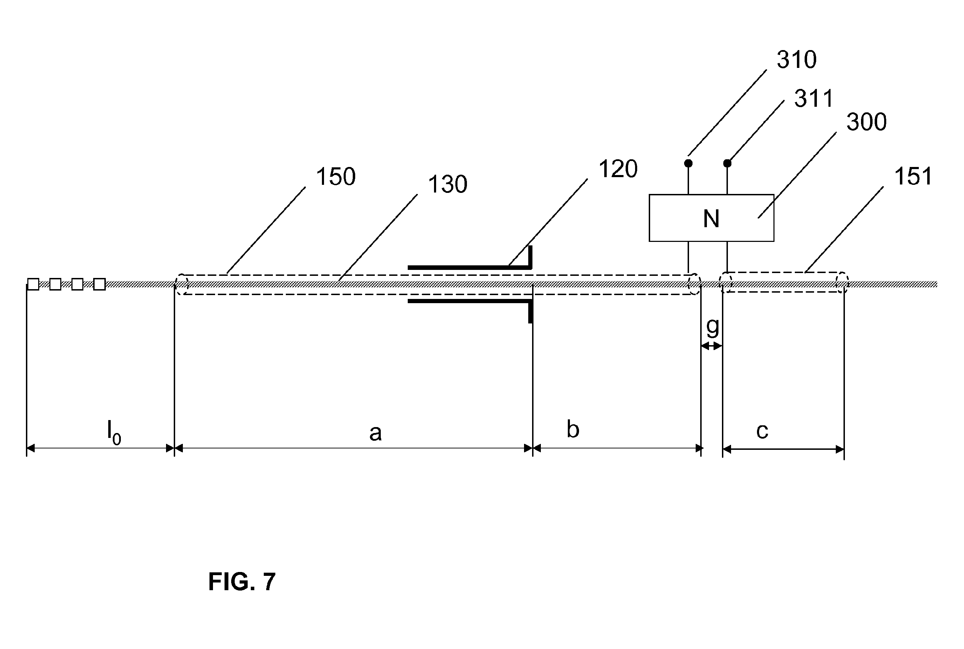

Fig 7 zeigt eine weitere Ausgestaltung der Ableitvorrichtung mit einem zweiten Sheathabschnitt 151 der Länge c mit einem Spalt der Länge g zur Ableithülle der Ableitvorrichtung 150. Der Ableitvorrichtung 150 wird mindestens ein zweiter Sheath 151 nachgeschaltet, wobei zwischen jedem dieser Sheath(abschnitte) 151 jeweils elektrische Netzwerke 300 eine elektrische Kopplung der Ableitvorrichtung mit den einzelnen zweiten Sheathabschnitten 151 vornimmt. Die Netzwerke 300 sind ebenfalls mit RLC-Gliedern und/oder Übertragern realisiert, können aber auch Wellenleitern umfassen. Alle diese Komponenten sind einstellbar realisiert.

Diese Netzwerke 300 verkoppeln den Energieeintrag benachbarter Sheath 150/151 derart, dass die unerwünschte Erwärmung des Katheters vermieden oder reduziert wird. Die Netzwerke 300 verfügen ebenso über Anschlüsse 310, 311 für Energieabfuhr oder kompensierende Energieeinkopplung mit Kontaktierungsmöglichkeiten an Gegenpole bzw. Anschluss eines Generators, wie unter Figurenbeschreibung 6 genannt. Die zusätzlichen Sheath 151 sind getrennt verschiebbar, der Abstand g ist also variabel. Ebenso sind die Längen c einstellbar eines jeden Sheath 151 einstellbar, beispielsweise ähnlich oder gleich gestaltet wie das weiter oben erwähnte Teleskop.

Zusätzlich kann jede der dargestellten Ausführungsformen eine Einheit 400 aufweisen, welche ausgelegt ist, die Erfassung und Verarbeitung von Sensorsignalen und die Steuerung der oben genannten variablen Komponenten - wie beispielsweise einstellbare Gegenpole, einstellbare Hochfrequenzgeneratoren 430 inklusive PLL für den Phasenbezug zum Hochfrequenzsystem des Scanners, Netzwerke 220 und 300 und Abstände b, c und g - sicherzustellen und zu optimieren.

Dazu sind der Anwendungskatheter 130 und/oder die Ableitungsvorrichtung 150 und/oder die Sheath(abschnitte) 151 und/oder die Schleuse mit Sensoren 420, 421 oder 422 bestückt, die den Effekt der Energieeinkopplung wahrnehmen. Dies äußert sich in Temperaturanstieg an Elektrodenpolen 140 des Anwendungskatheters 130, erhöhten Stromflüssen durch die Leiter des Anwendungskatheters 130 und/oder erhöhte E-Felder. Dementsprechend kann es sich bei den Sensoren 420, 421 und 422 um Temperatur-, B-Feld-/Stromflusssensoren oder E-Feldsensoren handeln Die Sensoren geben ihre Signale an eine Einheit zur Erfassung und Verarbeitung 400 weiter. Die Signale werden dabei so geleitet, dass sie nicht von den Feldern des Scanners gestört werden. Maßnahmen hierfür sind beispielsweise Schirmungen, optische/akustische Links oder Funk auf einer Scanner-fremden Frequenz. Diese Verarbeitungseinheit 400 steuert dann die genannten variablen Komponenten im Aufbau dahingehend, dass die Erwärmung bzw. deren Verursacher Stromfluss und E-Felder minimiert werden. Dazu setzt diese Verarbeitungseinheit 400 einen Regler ein. Die Stellglieder sind dabei entsprechende Aktuatoren (z. B. Motoren die die notwendigen mechanischen Veränderungen umsetzen) oder optische und/oder akustische Anzeigen, die dem Bediener signalisieren, in welche Richtung er die Elemente zu stellen hat.

Zur Unterstützung der Platzierung im Körper ist Ableitvorrichtung 150 mit Mitteln versehen, welche es in der MRT-Bildgebung sichtbar machen, wie beispielsweise durch eine Tracking-Spule oder durch eine Kontrastmittelfüllung der Ableithülle aus Gadolinium oder Gadoliniumverbindungen oder superparamagnetischen Eisenoxid-Partikeln.

Zur Stabilisierung der Lage des Anwendungskatheters 130 und zum Erreichen der therapeutisch relevanten Zielorte ist die Ableitvorrichtung 150 mit einer geeigneten Vorformung versehen. Weiterhin kann die Ableitvorrichtung 150 zur Unterstützung der Manipulation Anwendungskatheters 130 mit einer gleitfähigen Innenbeschichtung versehen. Zur Vermeidung von Bildartefakten in der MRT-Bildgebung sind die elektrisch leitenden Teile -wie die Schirmung der Ableitvorrichtung 130 - insbesondere im distalen Bereich aus Materialien mit geeigneter magnetischer Suszeptibilität. Geeignete Materialien wären z. B. Kupfer, Aluminium und/oder Graphit/Carbonfasern.

Bezugszeichenliste:

100: menschlicher oder tierischer Körper

110: Körperlumen, insbesondere ein Blutgefäß

120: Einführschleuse

130: Anwendungskatheter

| 140: | therapeutische oder diagnostische Katheterpole des Anwendungskatheters |
|---|---|
| 150: | Sheath (auch "Abschirmvorrichtung) |
| 151: | ein zweites Sheath der Länge c mit einem Spalt der Länge g zu ersten Sheath (Abschirmvorrichtung") |
| 200: | elektrischer Abgriff (auch "Anschlussmittel") am Sheath in der Entfernung b1 von der Einführschleuse |
| 210: | Anschluss an einen Gegenpol wie eine Vorrichtung zur Energieabfuhr oder kompensierenden Energieeinkopplung |
| 220: | zusätzliches Anpassungsnetzwerk |
| 300: | Koppelnetzwerk N zur Verkopplung der Abschirmvorrichtung und des zweiten Sheath |
| 310, 311: | Anschlüsse für Energieabfuhr oder kompensierende Energieeinkopplung mit Kontaktierungsmöglichkeiten an Gegenpole |
| 400: | Einheit zur Erfassung und Verarbeitung von Sensorsignalen und Steuerung der oben genannten Variablen Komponenten im Aufbau |
| 410: | Temperatursensor an den Katheterpolen des Anwendungskatheters |
| 420-422: | B-Feld- und/oder E-Feldsensoren |
| 430: | einstellbarer Generator (inkl PLL für Phasenbezug zum HF System des Scanners) |

**Patentansprüche**

1. Ableitvorrichtung mit einem proximalen, außerhalb des Körpers befindlichen Ende und einem distalen Ende, insbesondere geeignet für lang gestreckte, temporär im Körper befindliche medizinische Instrumente, insbesondere elektrophysiologische Katheter oder temporäre Elektrodenleitungen, umfassend:

   - eine Ableithülle, die sich vom proximalen zum distalen Ende der Ableitvorrichtung erstreckt; und
   - ein Lumen, welches sich vom proximalen zum distalen Ende erstreckt und in welchem das Instrument verschieblich geführt werden kann, wobei das Lumen von der Ableithülle umschlossen ist,

   **dadurch gekennzeichnet, dass**
   die Ableithülle zumindest teilweise aus elektrisch leitfähigem Material besteht und/oder die Ableithülle ein Ableitmittel bestehend aus elektrisch leitfähigem Material aufweist, so dass die Ableithülle dazu ausgebildet ist, durch elektromagnetische Strahlung induzierte elektrische Energie abzuführen oder abzuleiten.

2. Ableitvorrichtung nach Anspruch 1, **dadurch gekennzeichnet, dass** das elektrisch leitfähige Material eine Polymermatrix ist, in welche leitfähige Partikel eingebettet sind, wobei die leitfähigen Partikel vorzugsweise eine lang gestreckte Form aufweisen, besonders bevorzugt mit einem Längen- zu Breiten- beziehungsweise Durchmesser-Verhältnis von größer 2, besonders bevorzugt von größer 100.

3. Ableitvorrichtung nach Anspruch 2, **dadurch gekennzeichnet, dass** die Polymermatrix vorzugsweise Silikon, Polyurethan und/oder Peba umfasst und die leitfähigen Partikel Carbon, bevorzugt Carbon black und/oder Carbonfasern, Carbon-Nanopartikel, metallbeschichtete Carbonpartikel und/oder Carbon-Nanotubes, Leitruß und/oder Metallpartikel umfassen.

4. Ableitvorrichtung nach einem der Ansprüche 1 bis 3, **dadurch gekennzeichnet, dass** die Ableithülle einen ersten distalen Ableitabschnitt und einen zweiten proximalen Ableitabschnitt umfasst, wobei der zweite Ableitabschnitt relativ zum ersten Abschnitt verschieblich gelagert ist, um die Länge der Ableitvorrichtung und damit die Schutzlänge der Ableitvorrichtung so zu optimieren, dass die Schutzwirkung maximal ist.

5. Ableitvorrichtung nach Anspruch 4, **dadurch gekennzeichnet, dass** der zweite Ableitabschnitt eine Führung aufweist, welche den ersten Ableitabschnitt flüssigkeitsdicht verschieblich führt.

6. Ableitvorrichtung nach Anspruch 1, **dadurch gekennzeichnet, dass** das leitfähige Material aus einem Metall, vorzugsweise einem biokompatiblen Metall gebildet ist, besonders bevorzugt ein Metallgeflecht, eine Metallwendel oder ein Metallprofil ist, wobei jedes dieser Ableitungsmittel zumindest teilweise in die Ableithülle eingebettet ist.

7. Ableitvorrichtung nach einem der Ansprüche 1 bis 6, **dadurch gekennzeichnet, dass** die Ableithülle und/oder das Ableitmittel mindestens abschnittsweise galvanisch oder kapazitiv mit dem die Ableitvorrichtung umgebenden Körpergewebe elektrisch gekoppelt sind, um die durch elektromagnetische Strahlung induzierte elektrische Energie an das umgebende Körpergewebe abzuführen oder abzuleiten.

8. Ableitvorrichtung nach einem der Ansprüche 1 bis 6, **dadurch gekennzeichnet, dass** die Ableithülle und/oder das Ableitmittel mindestens abschnittsweise gegenüber dem die Ableitvorrichtung umgebenden Körpergewebe elektrisch entkoppelt sind und die Ableitvorrichtung am proximalen Ende ein Anschlussmittel aufweist, welches mit dem Ableitmittel elektrisch verbunden ist, um die durch elektromagnetische Strahlung induzierte elektrische Energie an einen außerhalb des Körpers befindlichen Gegenpol galvanisch oder kapazitiv abzuführen oder abzuleiten.

9. Ableitvorrichtung nach Anspruch 8, **dadurch gekennzeichnet, dass** das Anschlussmittel am proximalen Ende des zweiten Ableitabschnittes, wobei das zweite Ableitmittel einen elektrischen Konnektor vorzugsweise an seinem distalen Ende aufweist, welcher geeignet ist, die vom ersten Ableitungsabschnitt aufgenommene Energie aufzunehmen und diese ebenfalls über das Anschlussmittel abzuleiten.

10. Ableitvorrichtung nach einem der Ansprüche 8 oder 9, **dadurch gekennzeichnet, dass** der Gegenpol eine Vorrichtung zur Energieabfuhr ist, vorzugsweise ist diese Vorrichtung die Schirmung der Kabine des MR-Diagnosegerätes, der Hochfrequenzschirm des Scanners, der Schirm eines elektrophysiologischen Ablationsgerätes oder das Potential der Neutralelektrode des elektrophysiologischen Ablationssystems.

11. Ableitvorrichtung nach einem der Ansprüche 8 bis 10, **dadurch gekennzeichnet, dass** das Anschlussmittel zusätzlich oder alternativ zum Gegenpol mit einem in Amplitude und Phase einstellbaren Hochfrequenzgenerator zur kompensierenden Energieeinkopplung elektrisch verbunden ist.

12. Ableitvorrichtung nach einem der Ansprüche 8 bis 11, **dadurch gekennzeichnet, dass** zwischen Anschlussmittel, Gegenpol und/oder Hochfrequenzgenerator ein Anpassungsnetzwerk bestehend aus RLC-Gliedern und/oder Übertragern vorgesehen ist, welches die Optimierung der Energieabfuhr oder -ableitung sicherstellt.

13. System zur Behandlung des menschlichen Körpers bestehend aus einer Ableitvorrichtung nach einem der Ansprüche 1 bis 12 und einem lang gestreckten, temporär im Körper befindlichen medizinischen Instrument mit einem in den menschlichen Körper einführbaren ersten Bereich mit einem distalen Ende und einem außerhalb des Körpers befindlichen zweiten Bereich mit einem proximalen Ende, und mit mindestens einer galvanisch elektrischen leitenden Struktur, wobei das medizinische Instrument in der Ableitvorrichtung verschieblich gelagert ist, **dadurch gekennzeichnet, dass** die Ableitvorrichtung mindestens abschnittsweise den ersten Bereich des medizinischen Instrumentes umschließt.

14. System nach Anspruch 13, **dadurch gekennzeichnet, dass** der nicht umschlossene Abschnitt des medizinischen Instruments zwischen seinem distalen Ende und dem distalen Ende der Ableitvorrichtung kleiner als 1/4, vorzugsweise 1/10 der Wellenlänge einer einstrahlenden Hochfrequenz-Welle ist.

15. System nach einem der Ansprüche 13 oder 14, **dadurch gekennzeichnet, dass** die Ableitvorrichtung eine Arretierungseinrichtung umfasst, mit welcher das medizinische Instrument lösbar arretiert so werden kann, so dass ein Verschieben des medizinischen Instruments in der Ableitvorrichtung verhindert wird.

FIG. 1

FIG. 3

FIG. 2

FIG. 4

**FIG. 5**

EP 2 380 489 A1

**FIG. 6**

FIG. 7

EP 2 380 489 A1

**FIG. 8**

Europäisches
Patentamt

European
Patent Office

Office européen
des brevets

**EUROPÄISCHER RECHERCHENBERICHT**

Nummer der Anmeldung

EP 10 16 1071

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2005/110217 A1 (NANOSET LLC [US]; WANG XINGWU [US]; GREENWALD HOWARD J [US]; HELFER JE) 24. November 2005 (2005-11-24) <br> * Seite 16, Zeilen 20-23 * <br> * Seite 21, Zeilen 15-20 * <br> * Seite 26, Zeilen 5-24 * <br> * Seite 30, Zeilen 8-33 * <br> * Seite 31, Zeilen 11,12 * <br> * Seite 35, Zeile 6 - Seite 36, Zeile 29 * <br> * Seite 38, Zeilen 10-30 * <br> * Abbildungen 14,17,18,19A * <br> ----- | 1-3,6-8, 11,13,14 | INV. A61B5/00 |
| X | US 2008/097193 A1 (KARMARKAR PARAG V [US]) 24. April 2008 (2008-04-24) <br><br> * Absatz [0035] - Absatz [0047] * <br> * Absatz [0073] * <br> * Abbildungen 1-5,7,8 * <br> ----- | 1,4,5, 8-10, 12-14 | |
| X | US 2008/312636 A1 (MILLER DAVID [US] ET AL) 18. Dezember 2008 (2008-12-18) <br> * Absatz [0100] - Absatz [0104] * <br> * Abbildungen 2A-2C * <br> ----- | 1,4-7, 13-15 | |
| X | US 5 431 638 A (HENNIG WALTER S [US] ET AL) 11. Juli 1995 (1995-07-11) <br> * Spalte 1, Zeilen 29-51 * <br> * Spalte 2, Zeilen 48-58 * <br> ----- | 1-3,6-8, 10,13-15 | |
| X | US 2005/222658 A1 (HOEGH THOMAS B [US] ET AL) 6. Oktober 2005 (2005-10-06) <br> * Absatz [0055] - Absatz [0056] * <br> * Absatz [0059] * <br> * Abbildung 16 * <br> ----- <br><br> -/-- | 1,13 | |

RECHERCHIERTE SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| Berlin | 9. September 2010 | Völlinger, Martin |

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 10 16 1071

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 1 923 095 A2 (BIOTRONIK CRM PATENT AG [CH]) 21. Mai 2008 (2008-05-21)<br>* Absatz [0013] *<br>* Absätze [0039], [0040] *<br>* Absatz [0046] - Absatz [0052] *<br>----- | 1-3 | |
| A | US 2008/195186 A1 (LI BERNARD [US] ET AL) 14. August 2008 (2008-08-14)<br>* Absatz [0008] *<br>* Absatz [0035] *<br>* Absatz [0042] *<br>* Absatz [0045] - Absatz [0046] *<br>* Absatz [0055] - Absatz [0056] *<br>----- | 1-3 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Berlin | 9. September 2010 | Völlinger, Martin |

EPO FORM 1503 03.82 (P04C03)

## ANHANG ZUM EUROPÄISCHEN RECHERCHENBERICHT
### ÜBER DIE EUROPÄISCHE PATENTANMELDUNG NR.

EP 10 16 1071

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-09-2010

| Im Recherchenbericht angeführtes Patentdokument | Datum der Veröffentlichung | Mitglied(er) der Patentfamilie | Datum der Veröffentlichung |
|---|---|---|---|
| WO 2005110217 A1 | 24-11-2005 | KEINE | |
| US 2008097193 A1 | 24-04-2008 | CA 2575313 A1<br>EP 1786320 A2<br>JP 2008508043 T<br>WO 2006014966 A2 | 09-02-2006<br>23-05-2007<br>21-03-2008<br>09-02-2006 |
| US 2008312636 A1 | 18-12-2008 | US 2008312609 A1<br>US 2008312546 A1<br>WO 2008157227 A1 | 18-12-2008<br>18-12-2008<br>24-12-2008 |
| US 5431638 A | 11-07-1995 | CA 2065711 A1<br>DE 69222558 D1<br>DE 69222558 T2<br>EP 0508453 A1 | 11-10-1992<br>13-11-1997<br>26-02-1998<br>14-10-1992 |
| US 2005222658 A1 | 06-10-2005 | AT 476219 T<br>EP 1740260 A1<br>WO 2005102445 A1 | 15-08-2010<br>10-01-2007<br>03-11-2005 |
| EP 1923095 A2 | 21-05-2008 | KEINE | |
| US 2008195186 A1 | 14-08-2008 | EP 2125103 A1<br>WO 2008100840 A1 | 02-12-2009<br>21-08-2008 |

EPO FORM P0461

Für nähere Einzelheiten zu diesem Anhang : siehe Amtsblatt des Europäischen Patentamts, Nr.12/82

**EP 2 380 489 A1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6944489 B
- US 20030144720 A1
- US 20030144721 A1
- US 20050288751 A1
- US 20050288752 A1
- US 20050288754 A1
- US 20050288756 A1
- EP 0884024 B1
- US 20060200218 A1